(19) Europäisches Patentamt
European Patent Office
Office européen des brevets

(11) Veröffentlichungsnummer : **0 474 590 A2**

(12) # EUROPÄISCHE PATENTANMELDUNG

(21) Anmeldenummer : **91810614.7**

(22) Anmeldetag : **06.08.91**

(51) Int. Cl.$^5$ : **C07H 15/04,** A01N 43/16

(30) Priorität : **13.08.90 CH 2626/90**

(43) Veröffentlichungstag der Anmeldung :
**11.03.92 Patentblatt 92/11**

(84) Benannte Vertragsstaaten :
**AT CH DE FR IT LI**

(71) Anmelder : **CIBA-GEIGY AG**
**Klybeckstrasse 141**
**CH-4002 Basel (CH)**

(71) Anmelder : **Schweizerische Eidgenossenschaft handelnd durch die Eidg.Forschungsanstalt für Obst-, Wein- und Gartenbau Wädenswil**
**CH-8820 Wädenswil (CH)**

(72) Erfinder : **Ernst, Beat, Dr.**
**Obere Egg 6**
**CH-4312 Magden (CH)**
Erfinder : **Heneghan, Michael, Dr.**
**Steinrebenstrasse 43**
**CH-4153 Reinach (CH)**
Erfinder : **Wagner, Beatrice**
**St. Johanns-Ring 28**
**CH-4056 Basel (CH)**
Erfinder : **Boller, Ernst, Dr.**
**Büelenebnetstrasse 27**
**CH-8820 Wädenswil (CH)**
Erfinder : **Hurter, Jakob, Dr.**
**Hintere Bergstrasse 18**
**CH-8942 Oberrieden (CH)**
Erfinder : **Städler, Erich, Dr.**
**Palmisacker**
**CH-8821 Schönenberg (CH)**

(54) **Taurinderivate, Verfahren zu deren Herstellung und deren Verwendung.**

(57) Verbindung der Formel I

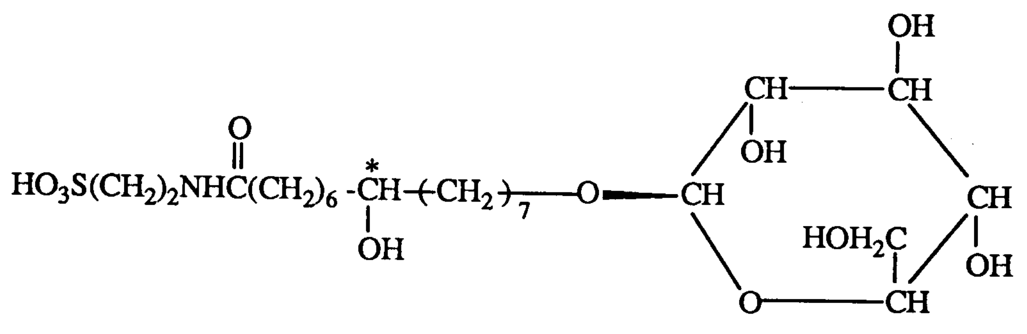

(I),

in Form ihrer optischen Isomeren (8R) oder (8S) oder deren Gemische, sowie ihre Alkalimetall- und Erdalkalimetallsalze eignen sich zum Schützen von Kirschen gegen den Befall mit Kirschenfliegen.

EP 0 474 590 A2

Die Erfindung betrifft N-[15-(Glucopyranosyloxy)-8-hydroxypentadecanoyl]-taurin und dessen Alkali- oder Erdalkalimetallsalze, ein Verfahren zu deren Herstellung und deren Verwendung zum Schützen von Kirschen gegen den Befall mit Kirschenfliegen.

In der EP-A-0 230 397 wird die Isolierung von N-[15-(β-Glucopyranosyloxy)-8-hydroxypalmitoyl]-taurin aus den nach der Eiablage auf der Oberfläche von Kirschfrüchten abgesonderten Exkrementen der weiblichen Kirschenfliege Rhagoletis cerasi L. beschrieben. Das Taurinderivat erwies sich als Pheromon, das weitere Eiablagen in der gleichen Frucht weitgehend verhindert. Bei nur sehr geringen Auftragungsmengen können Kirschfrüchte mit diesem Pheromon daher wirksam vor dem Befall mit Kirschenfliegen geschützt werden.

Das Taurinderivat enthält zwei chirale Zentren am C(8) und C(15). In der EP-A-0 352 230 wird die Synthese von 4 optischen Isomeren beschrieben, die alle wirksam sind. Die Synthese ist sehr aufwendig.

Es wurde gefunden, dass die hohe Wirksamkeit bei nur geringen Auftragsmengen überraschend weitgehend erhalten bleibt, wenn das chirale Zentrum am C(15) mit dem Ersatz der Methylgruppe durch Wasserstoff aufgehoben wird. Die Synthese dieser Verbindungen gestaltet sich wesentlich einfacher.

Ein Gegenstand der Erfindung ist eine Verbindung der Formel I

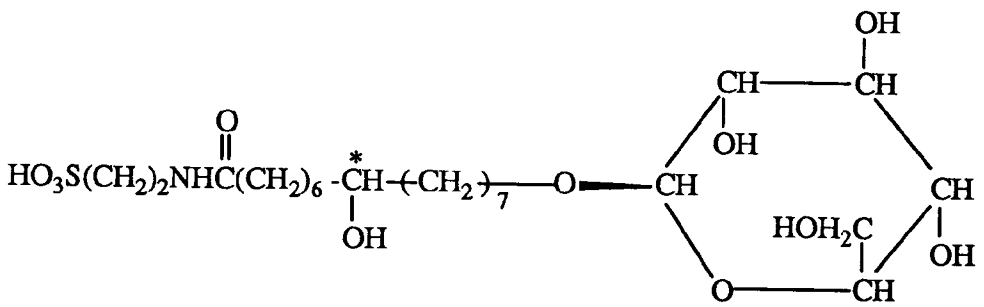

(I),

in Form ihrer optischen Isomeren (8R) oder (8S) oder deren Gemische, sowie ihre Alkalimetall- und Erdalkalimetallsalze.

Als Alkali- und Erdalkalimetallsalze kommen z.B. Li-, Na-, K-, Mg und Ca-Salze in Frage. Bevorzugt sind die Na-, K- und Mg-Salze.

Ein weiterer Gegenstand der Erfindung ist ein Verfahren zur Herstellung der Verbindung der Formel I, dadurch gekennzeichnet, dass man

a) eine Verbindung der Formel II

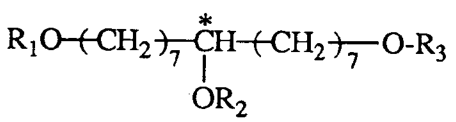

(II),

worin * die optischen Isomeren (R) und (S) bedeutet, und $R_1$, $R_2$ und $R_3$ unabhängig voneinander für eine abspaltbare Schutzgruppe stehen, mit D-Fluorglucopyranose, deren OH-Gruppen in den 2-, 3-, 4- und 6-Stellungen mit abspaltbaren Schutzgruppen geschützt sind, in Gegenwart von $BF_3$ als Diäthylätherat zu einer Verbindung der Formel III

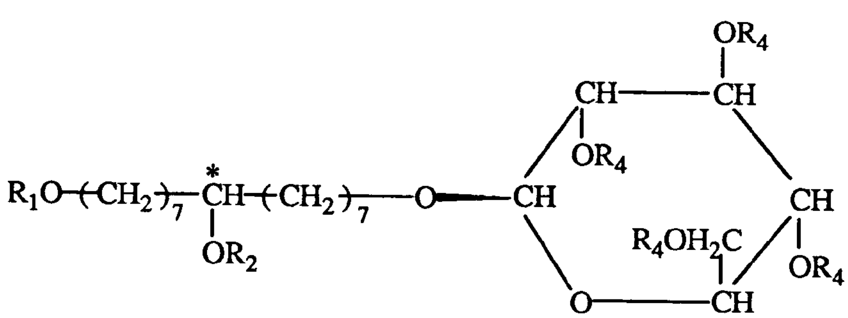

(III)

umsetzt, wobei $R_4$ eine Schutzgruppe bedeutet,

b) danach die Schutzgruppe $R_1$ entfernt und die erhaltene $-CH_2OH$-Gruppe zur Herstellung einer Verbindung der Formel IV

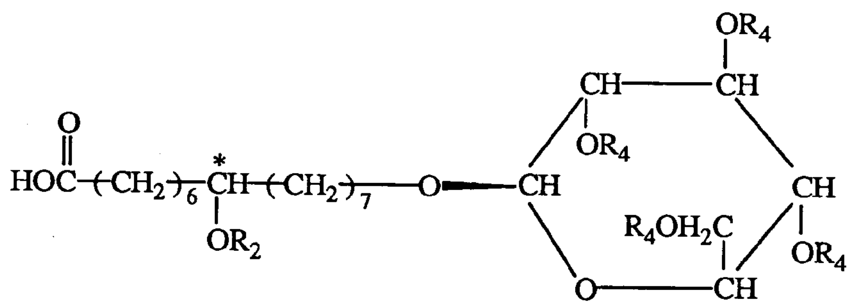

(IV)

oxidiert,

c) die Verbindung der Formel IV in einen zur Knüpfung einer Peptidbindung aktivierten Ester überführt und den aktivierten Ester mit Taurin oder seinen Alkali- oder Erdalkalimetallsalzen zu einer Verbindung der Formel V

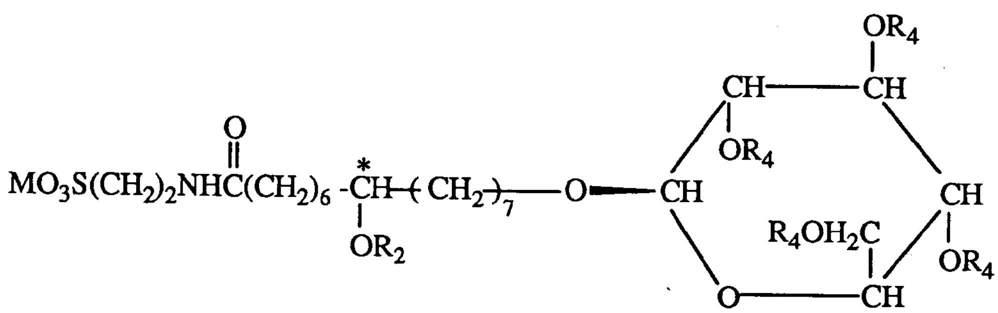

(V)

umsetzt, worin M Wasserstoff, ein Alkali- oder Erdalkalimetallkation bedeutet, umsetzt, und darauf

d) aus den Verbindungen der Formel V die Schutzgruppen $R_2$ und $R_4$ abspaltet und durch Wasserstoff ersetzt.

$R_1$, $R_2$ und $R_3$ können gleiche oder verschiedene Schutzgruppen sein. Hauptsächlich kommen solche Schutzgruppen in Frage, wie sie im allgemeinen in der Synthese von Kohlehydraten verwendet werden. Beispiele für Schutzgruppen sind Tri-($C_1$-$C_8$-alkyl)-silyl, ein mit $C_1$-$C_4$-Alkoxy substituiertes Phenyl, unsubstituiertes oder mit $C_1$-$C_4$-Alkyl substituiertes Benzyl, Diphenylmethyl oder Trityl, oder $C_1$-$C_8$-Acyl.

Beispiele für Trialkylsilyl sind Trimethyl-, Triäthyl-, Tripropyl-, Tributyl-, Isopropyl-dimethyl-, t-Butyldimethyl-, Hexyl-dimethyl-, Octyl-dimethyl- und (1,1,2,2-Tetramethyläthyl)-dimethylsilyl.

Bei der Alkoxyphenylgruppe kann es sich um

$$(C_1\text{-}C_4\text{-Alkoxy})_{1\text{-}5}\text{—phenyl}$$

handeln. Die Alkoxygruppe kann linear oder verzweigt und z.B. n-Butoxy, t-Butoxy, n- und i-Propoxy, Aethoxy und besonders Methoxy sein. Einige Beispiele sind p-Methoxyphenyl, 2,4-, 2,6-und 3,4-Dimethoxyphenyl und 3,4,5-Trimethoxyphenyl.

Die Benzyl-, Diphenylmethyl- und Tritylgruppe kann mit linearem oder verzweigtem $C_1$-$C_4$-Alkyl substituiert sein, z.B. mit Methyl, Aethyl, n- und i-Propyl und n-, i- und t-Butyl. Beispiele sind p-Methylbenzyl, 2,6-, 2,4-, 3,4-Dimethylbenzyl und 3,4,5-Trimethylbenzyl.

Das Acyl kann ein Rest der Formel R-CO- sein, worin R z.B. Wasserstoff oder lineares oder verzweigtes $C_1$-$C_8$-, besonders $C_1$-$C_6$-Alkyl,$C_5$- oder $C_6$-Cycloalkyl, Phenyl oder Benzyl ist. Beispiele für R sind Methyl, Aethyl und die Isomeren von Propyl, Butyl, Pentyl, Hexyl, Heptyl und Octyl.

In einer Ausführungsform sind $R_1$, $R_2$ und $R_3$ voneinander verschieden und besonders sind $R_1$ Tri-($C_1$-$C_8$-alkyl)silyl, $R_2$ $C_1$-$C_8$-Acyl und $R_3$ $C_1$-$C_4$-Alkoxyphenyl, oder unsubstituiertes oder mit $C_1$-$C_4$-Alkyl substituiertes Benzyl, Diphenylinethyl oder Trityl. Besonders bevorzugt sind $R_1$ t-Butyldimethylsilyl oder (1,1,2,2-Tetramethyläthyl)dimethylsilyl, $R_2$ Acetyl und $R_3$ p-Methoxyphenyl.

Die Schutzgruppen können nach bekannten Methoden eingeführt und abgespalten werden.

Die Verbindungen der Formel II können nach allgemein bekannten Verfahren hergestellt werden, wobei eine Herstellungsmethode in den Beispielen 1-5 beschrieben ist. Die Herstellung von 8-(p-Methoxyphenoxy)-octanol (siehe Beispiel 4) ist in den Beispielen 13 bis 15 der EP-A-0 352 230 beschrieben.

Die OH-Gruppen der α-D-Fluorglucopyranose bei der Verfahrensstufe a) sind bevorzugt mit einer Acylgruppe $R_4$ geschützt. $R_4$ steht im allgemeinen für $C_1$-$C_8$-Acyl wie Formyl, Acetyl, Propionyl, Benzoyl oder Pivaloyl. Bevorzugt steht $R_4$ für Pivaloyl. Die Reaktion wird bevorzugt in einem Halogenkohlenwasserstoff (z.B. $CH_2Cl_2$ oder $CHCl_3$) als Lösungsmittel und in Gegenwart von $BF_3$-Diäthylätherat durchgeführt.

Die Oxidation bei der Verfahrensstufe b) wird bevorzugt mit einem Cer(IV)-Salz (z.B. Cerammoniumnitrat) in Gegenwart von Wasser/Acetonitril durchgeführt.

Aktivierte Ester zur Knüpfung einer Peptidbindung, wie sie in der Verfahrensstufe c) verwendet werden, sind für Peptidsynthesen allgemein bekannt und z.B. in R. Geiger, Modern Methods in Protein Chemistry 2 (1985) 399. beschrieben. Bevorzugt sind Ester mit einem Dicarbonsäureimid-N-oxy-Rest in der Estergruppe, z.B. Hydroxysuccinimid-N-oxy.

Die Methoden der Abspaltung von Schutzgruppen bei der Verfahrensstufe d), die von der Art der Schutzgruppen abhängen, sind allgemein bekannt und z.B. in T.W. Greene, Protective Groups in Organic Synthesis, John Wiley & Sons, 1981 beschrieben.

Ein weiterer Gegenstand sind Verbindungen der Formel II. Für $R_1$, $R_2$ und $R_3$ gelten die zuvor beschriebenen Bevorzugungen.

Die Verbindungen der Formel I zeigen bei der elektrophysiologischen Prüfung biologische Aktivität und bei Verhaltensversuchen ausgezeichnete Wirkungen, selbst bei sehr geringen Auftragungsmengen.

Die erfindungsgemässen Wirkstoffe der Formel I sind stabile Verbindungen, bei deren Handhabung keine besonderen Vorsichtsmassnahmen notwendig sind.

Die Verbindungen der Formel I sind somit hochaktive Wirkstoffe, welche sich bei geeigneten Aufwandmengen hervorragend selektiv zum Schutz der Kirschfrüchte vor dem Befall mit Kirschenfliegen eignen, ohne dass die Früchte, Bäume oder die umgebende Flora oder Fauna geschädigt werden.

Die Erfindung betrifft auch Mittel zur Abwehr von Kirschenfliegen, dadurch gekennzeichnet, dass es neben Träger- und/oder anderen Zuschlagstoffen als Wirkstoff eine Verbindung der Formel I oder deren Gemische enthält.

Die Verbindungen der Formel I werden in unveränderter Form oder vorzugsweise zusammen mit den in der Formulierungstechnik üblichen Hilfsmitteln eingesetzt und können daher beispielsweise zu emulgierbaren Konzentraten, direkt versprüh- oder verdünnbaren Lösungen, verdünnten Emulsionen, Spritzpulvern, löslichen Pulvern, Stäubemitteln, Granulaten, auch Verkapselungen in polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, das heisst die den Wirkstoff der Formel I, beziehungsweise Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, zum Beispiel durch inniges Vermischen und/oder Vermahlen der Wirkstoffe mit Streckmitteln, wie beispielsweise mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$ von Alkylbenzolen wie Xylolgemische oder alkylierte Naphthaline, aliphatische oder cycloaliphatische Kohlenwasserstoffe, wie Cyclohexan, Paraffine oder Tetrahydronaphthalin, Alkohole wie Aethanol, Propanol oder Butanol, und Glykole sowie deren Aether und Ester, wie Propylenglykol, Dipropylenglykoläther, Aethylenglykol, Aethylenglykolmonomethyl- oder -äthyläther, Ketone, wie Cyclohexanon, Isophoron oder Diacetanolalkohol, starke polare Lösungsmittel, wie N-Methyl-2-pyrrolidon, Dimethylsulfoxid oder Dimethylformamid oder Wasser, Pflanzenöle, wie Raps-, Rizinus-, Kokosnuss- oder Sojaöl; gegebenenfalls auch Silikonöle.

Als feste Trägerstoffe, beispielsweise für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht sorptive Trägermaterialien Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und-/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie die Natrium- oder Kalium-Salze der Oel- oder Stearinsäure, oder von natürlichen Fettsäuregemischen, die beispielsweise aus Kokosnuss- oder Tallöl gewonnen werden können. Ferner sind als Tenside auch die Fettsäuremethyl-taurin-salze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, beispielsweise das Natrium- oder Calcium-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind zum Beispiel die Natrium-, Calcium- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie zum Beispiel Salze des Phosphorsäureesters eines p-Nonylphenol-(4-14)-Aethylenoxid-Adduktes oder Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkyrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quartemäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, beispielsweise das Stearyltrimethylammoniumchlorid oder das Benzyl-di-(2-chloräthyl)-äthylammoniumbromid.

Die in der Formulierungstechnik gebräuchlichen Tenside sind beispielsweise in folgenden Publikationen beschrieben:

"Mc Cutcheon's Detergents and Emulsifiers Annual", Mc Publishing Corp., Glen Rock, NJ, USA, 1988",
H. Stache, "Tensid-Taschenbuch", 2. Aufl., C. Hanser Verlag München, Wien 1981.
M. and J. Ash. "Encyclopedia of Surfactants", Vol. I-III, Chemical Publishing Co., New York, 1980- 1981.

Die erfindungsgemässen Mittel enthalten in der Regel 0,1 bis 99 %, insbesondere 0,1 bis 95 % Wirkstoff der Formel I oder Kombinationen dieses Wirkstoffs mit andern Insektiziden oder Akariziden, 1 bis 99,9 % eines festen oder flüssigen Zusatzstoffes und 0 bis 25 %, insbesondere 0,1 bis 25 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen. Typische Anwendungskonzentrationen liegen zwischen 0,01 und 1000 ppm, vorzugsweise zwischen 0,1 und 500 ppm. Die Aufwandmengen pro Hektar betragen im allgemeinen 20 bis 100 mg Wirkstoff pro Kirschbaum, vorzugsweise 40 bis 100 mg pro Kirschbaum.

Insbesondere setzen sich bevorzugte Formulierungen folgendermassen zusammen:
(% = Gewichtsprozent)

Emulgierbare Konzentrate:

| | |
|---|---|
| Aktiver Wirkstoff: | 1 bis 90 %, bevorzugt 5 bis 20 % |
| oberflächenaktives Mittel: | 1 bis 30 %, vorzugsweise 10 bis 20 % |
| flüssiges Trägermittel: | 5 bis 94 %, vorzugsweise 70 bis 85 % |

Stäube:

| | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |

Suspension-Konzentrate:

| | |
|---|---|
| Aktiver Wirkstoff: | 5 bis 75 %, vorzugsweise 10 bis 50 % |
| Wasser: | 94 bis 24 %, vorzugsweise 88 bis 30 % |
| oberflächenaktives Mittel: | 1 bis 40 %, vorzugsweise 2 bis 30 % |

Benetzbare Pulver:

| | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermaterial: | 5 bis 95 %, vorzugsweise 15 bis 90 % |

Granulate:

| | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 % |

Die Mittel können auch weitere Zusätze wie Stabilisatoren, z.B. gegebenenfalls epoxidierte Pflanzenöle (epoxidiertes Kokosnussöl, Rapsöl oder Sojaöl), Entschäumer, z.B. Silikonöl, Konservierungsmittel, Viskositätsregulatoren, Bindemittel, Hafmittel sowie Dünger oder andere Wirkstoffe zur Erzielung spezieller Effekte enthalten.

Weitere Gegenstände der Erfindung sind ein Verfahren zum Schützen von Kirschen gegen Befall mit Kirschenfliegen, dadurch gekennzeichnet, dass man auf der Kirschfrucht eine wirksame Menge eines Wirkstoffs der Formel I aufbringt sowie die Verwendung von Verbindungen der Formel I oder deren Gemische zum Schützen von Kirschen gegen den Befall mit Kirschenfliegen.

Die nachfolgenden Beispiele veranschaulichen die Erfindung.

## Herstellungsbeispiele

### Beispiel H1:

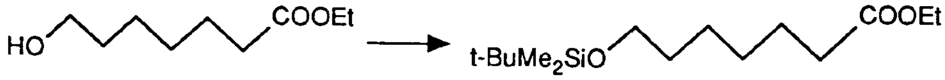

(1) (2)

16,42 g (94,36 mMol) (1) [Herstellung gemäss S. Canonica, M. Ferrari, M. Sisti OPPI BRIEFS 21, 253 (1989).] in 180 ml Dimethylformamid (DMF) werden bei Raumtemperatur (RT) mit 14,95 g (99,1 mMol) t-Butyldimethylsilylchlorid und 13,49 g (198,2 mMol) Imidazol versetzt und bei Raumtemperatur über Nacht gerührt. Zur Aufarbeitung wird das Reaktionsgemisch in 300 ml Methylenchlorid aufgenommen, je einmal mit Wasser und Sole gewaschen, mit $Na_2SO_4$ getrocknet. Nach Entfernen des Lösungsmittels am Rotationsverdampfer (Rotavap) wird der Rückstand (27,4 g) ohne weitere Reinigung in Beispiel H2 eingesetzt.

Abkürzungen:

Et = Aethyl
Me = Methyl
Bu = Butyl

### Beispiel H2:

(2) 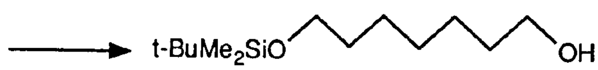

(3)

Der Rückstand (27,4 g) aus Beispiel H1 in 60 ml Diäthyläther wird während 30 Minuten zu 3,59 g (94,36 mMol) Lithiumaluminiumhydrid in 150 ml Diäthyläther bei 0°C getropft. Anschliessend wird das Reaktionsgemisch 1 Stunde am Rückfluss erhitzt. Zur Aufarbeitung wird das Reaktionsgemisch bei 0°C nacheinander mit 10,8 ml $H_2O$, 10,8 ml NaOH (15 %) und 32,4 ml $H_2O$ versetzt, 10 Minuten nachgerührt und über Diatomeenerde filtriert. Das Filtrat wird mit $MgSO_4$ getrocknet. Nach Entfernen des Lösungsmittels am Rotavap wird der Rückstand an Kieselgel (Hexan/Diäthyläther = 3:1 ) filtriert. Dabei werden 20,94 g (3) erhalten.
$^1$H-NMR (250 MHz, $CDCl_3$): 3,55 (t, J = 7, $-CH_2-OSi$).

### Beispiel H3:

(3) 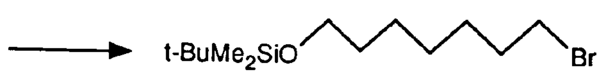

(4)

Zu 3,76 g (15,25 mMol) (3) in 24 ml Benzol werden bei Raumtemperatur tropfenweise 2,4 ml 1-Brom-2-N,N-trimethyl-propenyamin getropft. Anschliessend wird das Reaktionsgemisch 20 Minuten bei Raumtemperatur nachgerührt. Nach Entfernen des Lösungsmittels am Rotavap wird der Rückstand an Kieselgel (Hexan/Diäthyläther = 19:1) filtriert.
Dabei werden 4,53 g (4) erhalten.
$^1$H-NMR (250 MHz, $CDCl_3$): 3,55 (t, J = 7,0, $-CH_2O-$) ; 3,36 (t, J = 7,0, $-CH_2Br$).

Beispiel H4:

(4) +
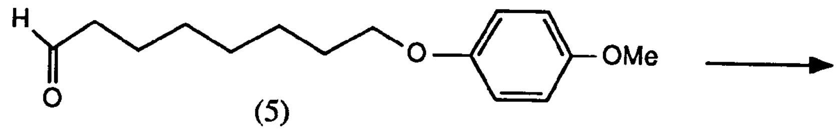

(5)

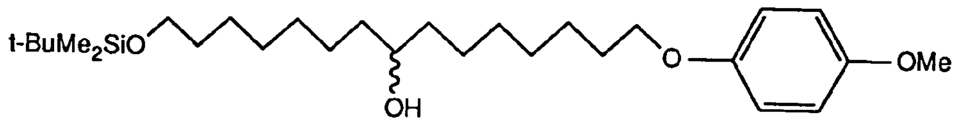

(6)

4,46 g (14,41 mMol) (4) in 15 ml Tetrahydrofuran werden bei Raumtemperatur mit 350 mg Magnesium umgesetzt. Nach Abkühlen des Reaktionsgemisches werden 3,61 g (14,41 mMol) (5) [Herstellung siehe Beispiele 13 bis 15, EP-A-0 352 230] in 10 ml Tetrahydrofuran unter Eiskühlung zugetropft. Anschliessend wird das Reaktionsgemisch auf Raumtemperatur erwärmt, 1 Stunde nachgerührt, mit 15 ml 2N $H_2SO_4$ versetzt und mit 50 ml Diäthyläther verdünnt. Dann wird die organische Phase mit $H_2O$ und Sole gewaschen, mit $Na_2SO_4$ getrocknet und das Lösungsmittel am Rotavap entfernt. Chromatographie des Rückstandes an Kieselgel (Pentan/Essigester = 8:1) ergibt 5,82 g (6).

$^1$H-NMR (250 MHz, $CDCl_3$): 3,90 (t, J = 7,0, $-CH_2O-$); 3,77 (s, OMe);3,60 (t, J = 7,0, $-CH_2O-$).

Beispiel H5:

(6)
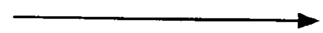

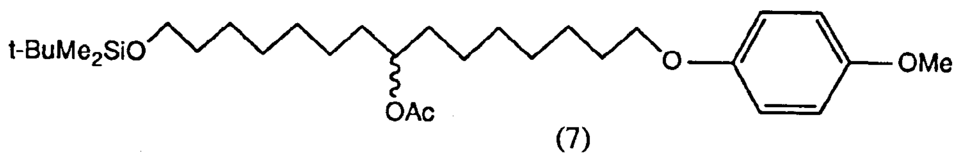

(7)

2,1 g (4,36 mMol) (6), 1,7 ml Essigsäureanhydrid und 2,8 ml Pyridin in 5 ml Methylenchlorid werden über Nacht bei Raumtemperatur gerührt. Dann wird das Reaktionsgemisch mit Essigsäureäthylester verdünnt, je einmal mit 1N HCl, $H_2O$, gesättigter $NaHCO_3$ und Sole gewaschen und mit $Na_2SO_4$ getrocknet. Nach Entfernen des Lösungsmittels am Rotavap wird der Rückstand (2,3 g) ohne weitere Reinigung direkt in Beispiel H6 eingesetzt.

$^1$H-NMR (500 MHz, $CDCl_3$): 3,85 (t, J = 7,0, $-CH_2O-$); 3,73 (s, OMe);3,55 (t, J = 7,0, $-CH_2O-$); 1,99 (s, OAc).

Abkürzung: Ac = Acetyl

Beispiel H6:

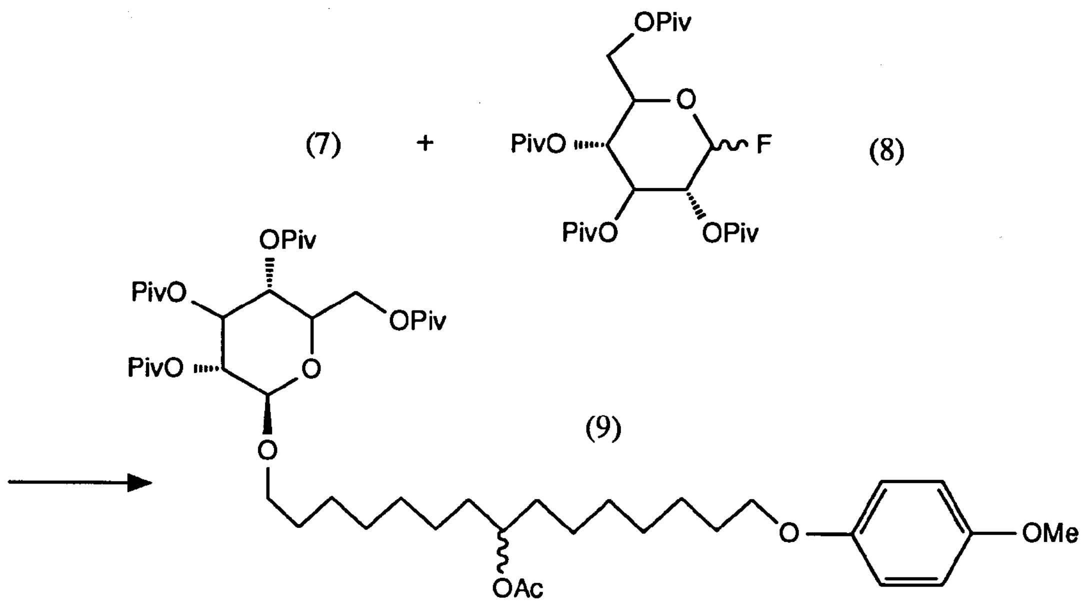

(7) + (8)

(9)

2,07 g (3,96 mMol) (7) und 2,05 g (3,96 mMol) 2,3,4,6-Tetrapivaloyl-D-fluorglucopyranose (8) in 4 ml Methylenchlorid werden bei Raumtemperatur während 3 Minuten mit 2,09 ml Bortrifluoriddiäthylätherat versetzt. Nach 30 Minuten wird mit Essigsäureäthylester verdünnt, je 1 Mal mit $H_2O$, gesättigter $NaHCO_3$ und Sole gewaschen, mit $Na_2SO_4$ getrocknet und eingeengt. Das Rohprodukt wird an Kieselgel (Petroleumbenzin 40-60°C/Essigsäureäthylester = 6:1 ) chromatographiert. Dabei erhält man 2,80 g (9).
$^1$H-NMR (500 MHz, $CDCl_3$): 4,48 (d, J = 8, anomeres H).
Abkürzung: Piv = Pivaloyl

Beispiel H7:

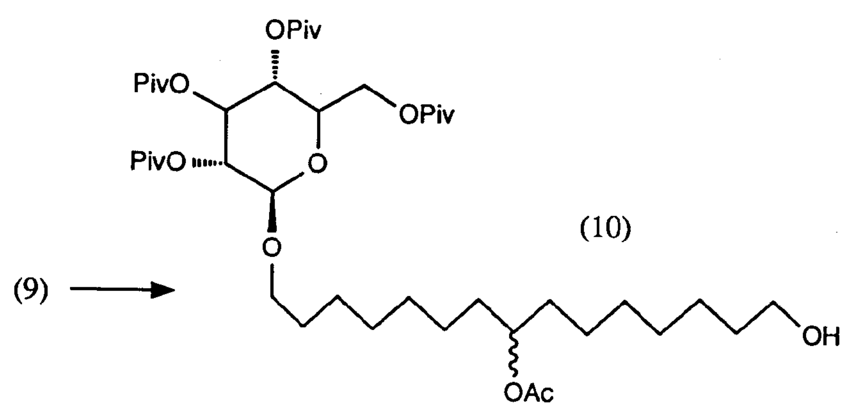

(9) (10)

2,0 g (2,20 mMol) (9) und 4,23 g Cerammoniumnitrat in 30 ml Acetonitril und 7,5 ml $H_2O$ werden bei 0°C 10 Minuten gerührt. Zur Aufarbeitung wird mit Diäthyläther verdünnt und je einmal mit $H_2O$, gesättigter $NaHCO_3$ und Sole gewaschen. Nach Trocknen der organischen Phase mit $Na_2SO_4$ wird das Lösungsmittel am Rotavap entfernt. Der Rückstand wird an Kieselgel (Petroleumbenzin 40-60°C/Essigsäureäthylester = 2:1 ) chromatographiert. Dabei erhält man 1,565 g ( 10).
$^1$H-NMR (300 MHz, $CDCl_3$): 4,49 (d, J = 8, anomeres H).

Beispiel H8:

(10)

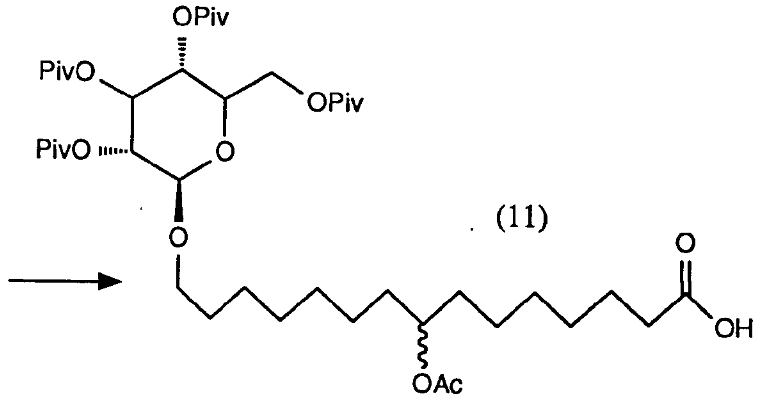

(11)

1,54 g (1,92 mMol) (10) in 20 ml Aceton werden bei 0°C mit 1,69 ml einer wässrigen 4N-$CrO_3$/$H_2SO_4$-Lösung (1:1) versetzt und 30 Minuten bei 0°C nachgerührt. Anschliessend wird mit Diäthyläther verdünnt, an Watte filtriert und je einmal mit $H_2O$ und Sole gewaschen. Nach Trocknen mit $Na_2SO_4$ wird das Lösungsmittel am Rotavap entfernt. Der Rückstand wird an Kieselgel (Petroleumbenzin/Essigsäureäthylester/Essigsäure = 75:22:3) chromatographiert. Dabei erhält man 1,24 g (11).
$^1$H-NMR (300 MHz, $CDCl_3$): 4,57 (d, J = 8, anomeres H).

Beispiel H9:

(11)

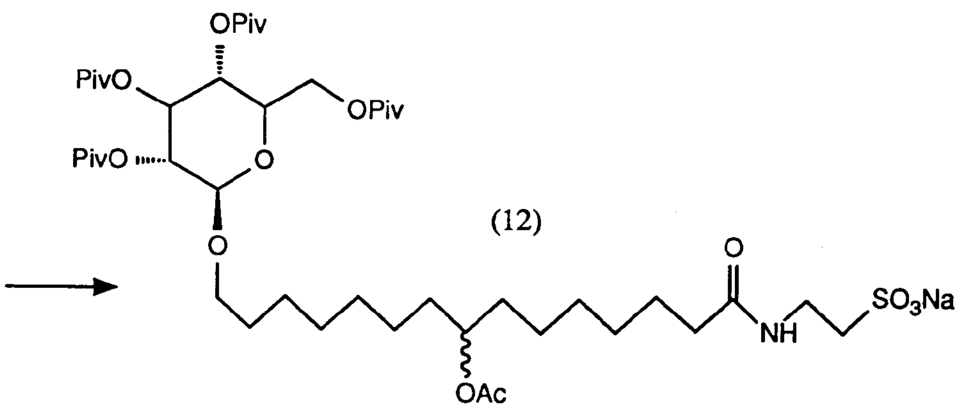

(12)

Ein Gemisch von 1,22 g (1,50 mMol) (11), 207 mg N-Hydroxysuccinimid und 402 mg Dicyclohexylcarbodiimid in 10 ml Dimethoxyäthan werden bei Raumtemperatur 17 Stunden gerührt. Anschliessend wird das Reaktionsgemisch filtriert, das Filtergut mit 10 ml Dimethoxyäthan nachgespült und das Filtrat eingeengt. Der Rückstand wird in 12 ml Methanol gelöst und bei Raumtemperatur mit 188 mg Taurin in 1,58 ml 1N NaOH und 4 ml Methanol versetzt. Nach 1,5 Stunden Rühren bei Raumtemperatur wird das Reaktionsgemisch eingeengt und an Kieselgel (Chloroform/Methanol = 4:1) chromatographiert. Dabei erhält man 1, 12 g (12).
$^1$H-NMR (300 MHz, $CDCl_3$): 4,50 (d, J = 8, anomeres H).

Beispiel H10:

(12)

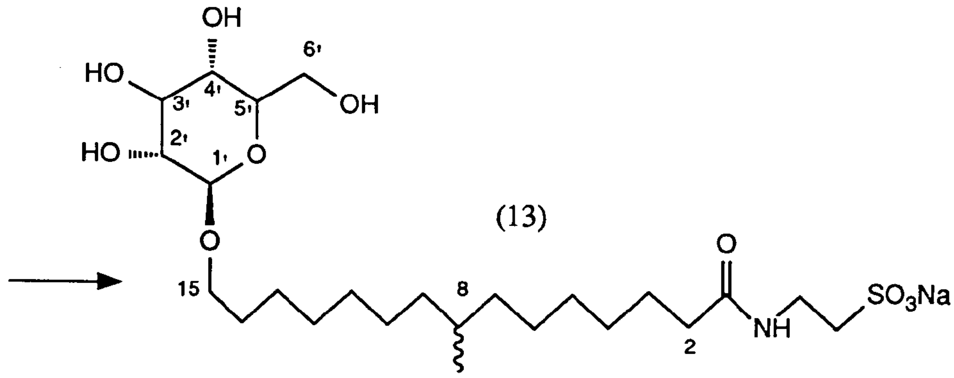

(13)

1,087 g (1,14 mMol) (12) in 10 ml Methanol und eine katalytische Menge Natrium werden im Bombenrohr bei 100°C 2 Stunden gerührt, Anschliessend wird das Reaktionsgemisch eingeengt und an Normalphasen-Kieselgel (Chloroform/Methanol = 1:1) und Umkehrphasen-Kieselgel (Acetonitril/$H_2O$) chromatographiert. Dabei erhält man 610 mg (13).

$^1$H-NMR (500 MHz, $CD_3OD$): 4,24 (d, J = 8, H-C(1')); 3,89 (dt, $J_1$ = 9, $J_2$ = 7, H-C(15)); 3,86 (dd, $J_1$ = 11,5, $J_2$ = 2, H-C(6')); 3,66 (dd, $J_1$ = 11,5, $J_2$ = 5,5, H-C(6')); 3,59 (t, J = 7, S-$CH_2$); 3,53 (dt, $J_1$ = 9,5, $J_2$ = 7, H-C(15)); 3,50 (m, H-C(8)); 3,40-3,20 (m, H-C(3'), H-C(4'), H-C(5')); 3,16 (dd, $J_1$ = 9, $J_2$ = 8, H-C(2')); 2,96 (t, I = 7, N-$CH_2$); 2,19 (t, J = 7,4, 2H-C(2)); 1,70- 1,25 (übrige aliphatische Protonen).

Formulierungsbeispiele

| Beispiel F1: Spritzpulver | a) | b) | c) |
|---|---|---|---|
| Wirkstoff gemäss Beispiel H10 | 25 % | 50 % | 75 % |
| Na-Ligninsulfonat | 5 % | 5 % | - |
| Na-Laurylsulfat | 3 % | - | 5 % |
| Na-Diisobutylnaphthalin-sulfonat | - | 6 % | 10 % |
| Octylphenolpolyäthylenglykol-äther (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle gut vennahlen. Man erhält Spritzpulver, die sich mit Wasser zu Suspensionen jeder gewünschten Konzentration verdünnen lassen.

Beispiel F2: Emulsions-Konzentrat

| | |
|---|---|
| Wirkstoff gemäss Beispiel H10 | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Beispiel F3: Stäubemittel | a) | b) |
|---|---|---|
| Wirkstoff gemäss Beispiel H10 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

Beispiel F4: Extruder-Granulat

| | |
|---|---|
| Wirkstoff gemäss Beispiel H10 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

Beispiel F5: Umhüllungs-Granulat

| | |
|---|---|
| Wirkstoff gemäss Beispiel H10 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

Beispiel F6: Suspensions-Konzentrat

| | |
|---|---|
| Wirkstoff gemäss Beispiel H10 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| Silikonöl in Form einer 75 %igen wässrigen Emulsion | 1 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Beispiel B1: Elektrophysiologische Prüfung

Die Prüfung auf biologische Aktivität der Verbindung gemäss Beispiel H10 erfolgt mittels elektrophysiologischen Ableitungen von torsalen Kontakt-Chemorezeptoren, wobei nach der von E. Städler beschriebenen Methode vorgegangen wird (E. Städler: Contact Chemoreception; Chemical Ecology of Insects, 1984, p. 3-35). Die zu messenden Proben bestehen aus 10 µl Lösung aufgelöst in 1 ml 10 mN Natriumchlorid-Lösung. 2 µl dieser verdünnten Lösung werden in eine Glas-Kapillarelektrode aufgesogen und zur Stimulierung 4 bis 8 Rezeptoren des Prothorax tarsus einer weiblichen Kirschenfliege verwendet. Die Verbindung zeigt biologische Aktivität im gemessenen Konzentrationsbereich von $5 \cdot 10^{-8}$ M bis $10^{-5}$ M.

Beispiel B2: Verhaltensprüfung

In 6 Versuchsserien wird eine Standardlösung von $10^{-3}$ mg Verbindung gemäss Beispiel H10 in 1 ml Methanol appliziert und die Verhinderung der Eiablage bestimmt. Pro Versuchseinheit werden auf einer standardisierten Plattform 12 Kirschenfliegenweibchen je 15 unbehandelte Kirschen, je 8 mit Verbindung H10 und je 7 mit einem anderen Wirkstoff behandelte Kirschen während 1 Stunde dargeboten. Registriert wird die Anzahl Landungen auf den einzelnen Kirschen, Einbohrversuche und erfolgreiche Eiablagen, die durch markierendes Kreisen der Weibchen nach beendeter Eiablage festgestellt werden.

Berechnung der biologischen Aktivität:

Nach Abschluss der Versuche werden die erfolgreichen Eiablagen pro Besuch auf jeder Behandlungsvariante berechnet. Mit diesem Zahlenmaterial wird der Diskriminierungskoeffizient (DC) zwischen behandelten und unbehandelten Kirschen wie folgt berechnet:

$$DC = \frac{\text{Eiablagen in Kontrolle} - \text{Eiablagen in Behandlung}}{\text{Eiablagen in Kontrolle} + \text{Eiablagen in Behandlung}} \times 100$$

Ein DC von +100 bedeutet, dass alle Eier in die unbehandelten Kontrollfrüchte abgelegt werden, während ein DC = 0 bedeutet, dass die Eier ohne Diskrimination gleichmässig in behandelte und unbehandelte Eiablagesubstrate abgelegt werden.

Ergebnis:

| Behandlung | absolutes Total aller Versuche | | | Durchschnittswerte der 6 Versuche | | |
|---|---|---|---|---|---|---|
| | Besuche | Eiablagen | DC | DC | ± | S.E. |
| Kontrolle | 408 | 221 | | | | |
| Verbindung H10 | 191 | 29 | 68,5 | 71,1 | ± | 8,73 |

Beurteilung:

Die Verbindung H10 zeigt im Verhaltenstest eine sehr gute biologische Aktivität.

**Patentansprüche**

1. Verbindung der Formel I

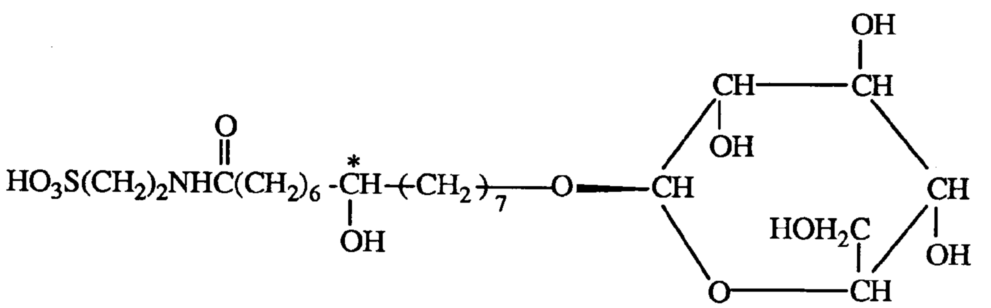

(I),

in Form ihrer optischen Isomeren (8R) oder (8S) oder deren Gemische, sowie ihre Alkalimetall- und Erdalkalimetallsalze.

2. Verbindung gemäss Anspruch 1, dadurch gekennzeichnet, dass es sich bei den Salzen um Natrium-, Kalium- und Magnesiumsalze handelt.

3. Verfahren zur Herstellung der Verbindung der Formel I nach Anspruch 1, dadurch gekennzeichnet, dass man
   a) eine Verbindung der Formel II

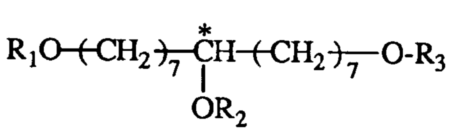

(II),

worin * die optischen Isomeren (R) und (S) bedeutet, und $R_1$, $R_2$ und $R_3$ unabhängig voneinander für eine abspaltbare Schutzgruppe stehen, mit D-Fluorglucopyranose, deren OH-Gruppen in den 2-, 3-, 4- und 6-Stellungen mit abspaltbaren Schutzgruppen geschützt sind, in Gegenwart von $BF_3$ als Diäthylätherat zu einer Verbindung der Formel III

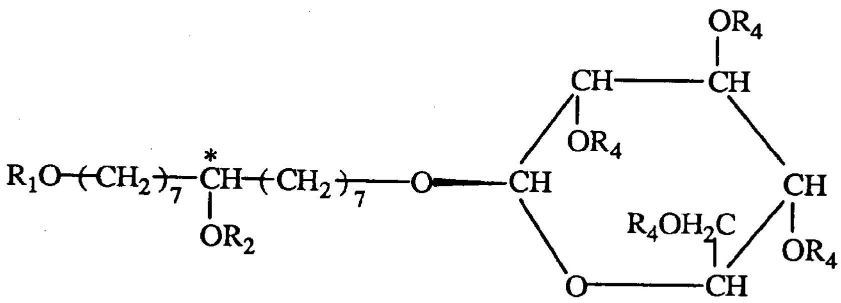

(III)

umsetzt, wobei $R_4$ eine Schutzgruppe bedeutet,

b) danach die Schutzgruppe $R_1$ entfernt und die erhaltene -$CH_2OH$-Gruppe zur Herstellung einer Verbindung der Formel IV

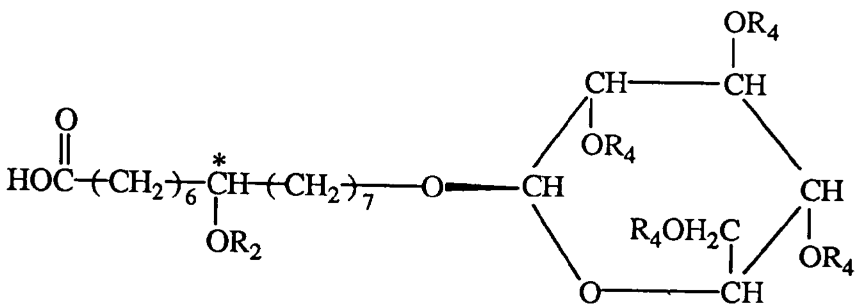

(IV)

oxidiert,

c) die Verbindung der Formel IV in einen zur Knüpfung einer Peptidbindung aktivierten Ester überführt und den aktivierten Ester mit Taurin oder seinen Alkali- oder Erdalkali-metallsalzen zu einer Verbindung der Formel V

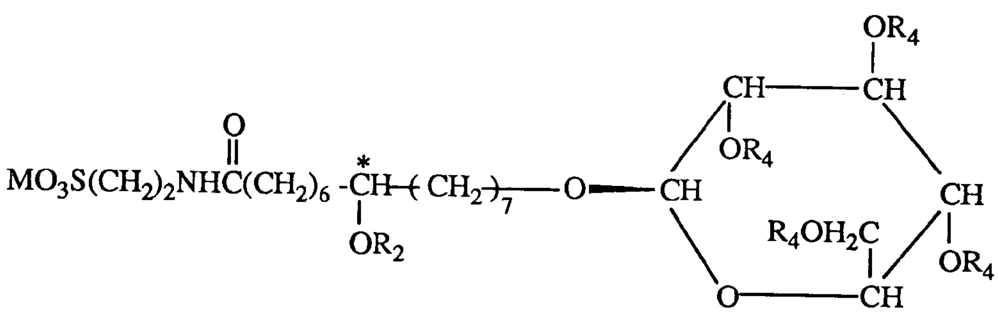

(V)

umsetzt, worin M Wasserstoff, ein Alkali- oder Erdalkalimetallkation bedeutet, umsetzt, und darauf

d) aus den Verbindungen der Formel V die Schutzgruppen $R_2$ und $R_4$ abspaltet und durch Wasserstoff ersetzt.

4. Verfahren nach Anspruch 3, dadurch gekennzeichnet, dass man in der Verfahrensstufe a) die Reaktion in einem Halogenkohlenwasserstoff als Lösungsmittel und in Gegenwart von $BF_3$-Diäthylätherat durchführt.

5. Verfahren nach Anspruch 3, dadurch gekennzeichnet, dass $R_1$, $R_2$ und $R_3$ voneinander verschieden sind.

6. Verfahren nach Anspruch 5, dadurch gekennzeichnet, dass $R_1$ Tri-($C_1$-$C_8$-alkyl)silyl, $R_2$ $C_1$-$C_8$-Acyl und $R_3$ $C_1$-$C_4$-Alkoxyphenyl, oder unsubstituiertes oder mit $C_1$-$C_4$-Alkyl substituiertes Benzyl, Diphenylmethyl oder Trityl sind.

7. Verfahren gemäss Anspruch 5, dadurch gekennzeichnet, dass $R_1$ t-Butyldimethylsilyl oder (1,1,2,2-Tetramethyläthyl)-dimethylsilyl, $R_2$ Acetyl und $R_3$ p-Methoxyphenyl sind.

8. Verfahren nach Anspruch 3, dadurch gekennzeichnet, dass die Oxidation in der Verfahrensstufe b) mit einem Cer(IV)-Salz in Gegenwart von Wasser durchgeführt wird.

9. Verfahren nach Anspruch 3, dadurch gekennzeichnet, dass es sich bei dem aktivierten Ester in der Verfahrensstufe c) um einen Ester mit einem Dicarbonsäureimid-N-oxy-Rest in der Estergruppe handelt.

10. Verfahren nach Anspruch 3, dadurch gekennzeichnet, dass $R_4$ in Formel III ein $C_1$-$C_8$-Acylrest ist.

11. Verfahren nach Anspruch 10, dadurch gekennzeichnet, dass $R_4$ für Pivaloyl steht.

12. Verbindungen der Formel II

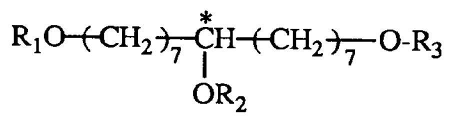

(II),

worin * die optischen Isomeren (R) und (S) bedeutet, und $R_1$, $R_2$ und $R_3$ unabhängig voneinander für eine abspaltbare Schutzgruppe stehen.

13. Verbindungen gemäss Anspruch 12, dadurch gekennzeichnet, dass $R_1$, $R_2$ und $R_3$ voneinander verschieden sind.

14. Verbindungen gemäss Anspruch 12, dadurch gekennzeichnet, dass $R_1$ Tri-($C_1$-$C_8$alkyl)silyl, $R_2$ $C_1$-$C_8$-Acyl und $R_3$ $C_1$-$C_4$-Alkoxyphenyl, oder unsubstituiertes oder mit $C_1$-$C_4$-Alkyl substituiertes Benzyl, Diphenylmethyl oder Trityl sind.

15. Verbindungen gemäss Anspruch 14, dadurch gekennzeichnet, dass $R_1$ t-Butyldimethylsilyl oder (1,1,2,2-Tetramethyläthyl)-dimethylsilyl, $R_2$ Acetyl und $R_3$p-Methoxyphenyl sind.

16. Mittel zur Abwehr von Kirschenfliegen, dadurch gekennzeichnet, dass es neben Träger- und/oder anderen Zuschlagstoffen als Wirkstoff eine Verbindung der Formel I oder deren Gemische gemäss Anspruch 1 enthält.

17. Mittel gemäss Anspruch 16, dadurch gekennzeichnet, dass der Wirkstoff in einer Menge von 0,1 bis 99 Gew.-% enthalten ist, bezogen auf das Mittel.

18. Mittel gemäss Anspruch 16, dadurch gekennzeichnet, dass der Träger ein $C_1$-$C_4$-Alkanol, Wasser oder eine der Mischungen davon ist.

19. Verfahren zum Schützen von Kirschen gegen Befall mit Kirschenfliegen, dadurch gekennzeichnet, dass man auf der Kirschfrucht eine wirksame Menge eines Wirkstoffs der Formel I gemäss Anspruch 1 aufbringt.

20. Verwendung von Verbindungen der Formel I oder deren Gemische gemäss Anspruch 1 zum Schützen von Kirschen gegen den Befall mit Kirschenfliegen.